# EUROPEAN PATENT APPLICATION

(11) **EP 3 287 266 A1**
(43) Date of publication of application: **28.02.2018**
(21) Application number: 17186572.8
(22) Date of filing: 17.08.2017
(51) Int. Cl.: B29D 11/00, A42B 3/22, A61F 9/02, B29C 45/14

(54) **A METHOD OF MANUFACTURING PHOTOCHROMIC LENS AND ARTICLE THEREOF**

(30) Priority: 17.08.2016 CN 201610680945
(71) Applicant: Dongguan Koda Optical Lens Co.,Ltd. DONGGUAN KODA OPTICAL LENS CO.,LTD., Dongguan, Guangdong 523000 (CN)
(72) Inventor: WU, Chih-Ming, Dongguan,, Guangdong 523000 (CN)
(74) Representative: Karakatsanis, Georgios

(57) **Abstract**

A method of manufacturing photochromic lens, which is characterized in that the method includes putting photochromic materials between the first layer of PC film and the second layer of PC film, glueing with optical cement, and then finalizing the design, thus forming a photochromic sheet (5) matching with the lateral sides of lens (4); placing the photochromic sheet (5) in the injection mold cavity of lens (4) in advance, which is placed in the lateral sides of lens (4); injecting the melted PC material into the cavity. Before curing, the PC material is at a high temperature of 260-280 , under which the PC material and photochromic sheet will fuse into one. After curing, a multi-layered photochromic lens is obtained. The present invention also provides a photochromic lens made by the above method. The present invention makes the combination of photochromic material and lens more stable and reliable, and not easy to fall off and lose effect even exposed to the sun and rain, so the photochromic effect can be maintained for a long time.

## Description

### Field of the Invention

The invention relates to a manufacturing art of lens, particularly to the manufacturing method of photochromic lens and the article of manufacture.

### Background of the Invention

Traditional helmet lens or ski lens is made of PC material, which doesn't have the photochromic effect. Considering that the lens is used under the environment with fierce and dazzling sunlight, it is necessary to take corresponding measures to provide lens with photochromic, sunshading and anti-dazzling effect. To this end, the most common practice is to add a layer of photochromic material. The traditional method of manufacturing photochromic lens is directly spraying a layer of photochromic material in the lateral sides of lens. As the photochromic material is sprayed to the lateral panel of lens directly, it will soon fall off and lose effect after being exposed to sun and rain, thus having a short service life. To sum up, the photochromic lens formed by direct spraying remains to be improved.

### Summary of Invention

To overcome the defects mentioned above, the present invention aims to provide a method of manufacturing photochromic lens, thus making the combination of photochromic material and lens more stable and reliable, and not easy to fall off and lose effect even exposed to the sun and rain, so the photochromic effect can be maintained for a long time. In addition, another object of the present invention is to provide a kind of photochromic lens.

The present invention adopts the technical scheme as follows: A method of manufacturing photochromic lens, which is characterized in that the method includes the following steps:
1) Put photochromic materials between the first layer of PC film and the second layer of PC film, glue with optical cement, and then finalize the design, thus forming a photochromic sheet matching with the lateral sides of lens;
2) Place the photochromic sheet formed in Step 1) in the injection mold cavity of lens in advance, which is placed in the lateral sides of lens;
3) Inject the melted PC material into the cavity. Before curing, the PC material is at a high temperature of 260-280 , under which the PC material and photochromic sheet will fuse into one. After curing, a multi-layered photochromic lens is obtained.

Preferably, in Step 1), the said photochromic material is a thin film structure sandwiched between the first layer of PC film and the second layer of PC film, or, the said photochromic material is a coating structure coated between the two layers of PC films.

Preferably, in Step 1), the said photochromic material is a kind of UV photochromic material.

Preferably, in Step 1), the thickness of the said first layer of PC film and second layer of PC film is between 0.1mm-0.5mm.

Preferably, in Step 1), the said photochromic material is placed between the first layer of PC film and the second layer of PC film, glued through optical cement and heated and bended to finalize the design. Also, it is feasible to heat and bend to finalize the design before gluing with optical cement.

A photochromic lens manufactured by the said method, including the lens and the photochromic sheet attached to the lateral sides of lens. It is characterized in that the said photochromic sheet is a four-layered structure sheet which is obtained by placing the photochromic material between the first layer of PC film and the second layer of PC film and gluing through optical cement. There are four layers, i.e., first layer of PC film, second layer of PC film, a layer of photochromic material and a layer of optical cement.

Preferably, the said photochromic material is a thin film structure sandwiched between the first layer of PC film and the second layer of PC film, or, the said photochromic material is a coating structure coated between the two layers of PC films.

Preferably, the said photochromic material is a kind of UV photochromic material.

Preferably, the thickness of the said first layer of PC film and second layer of PC film is between 0.1mm-0.5mm.

Wherein, the said lens is helmet lens or ski lens, or other similar lenses.

Preferably, as the first style, the said helmet lens is provided with a mounting hole on both ends, the inside of the hole is smooth, and the said photochromic sheet fully covers and fuses with the lateral sides of helmet lens;

Preferably, as the second style, the said helmet lens is provided with a mounting hole on both ends, the inside of the hole is of raised adjusting structure, and the said photochromic sheet fully covers and fuses with the lateral sides of helmet lens.

The present invention has the following advantages: First place the photochromic material between the first layer of PC film and the second layer of PC film, and glue with optical cement to form photochromic sheet; then by way of intramode injection molding method, the PC lens fuses with the photochromic sheet under high temperature in the process of curing, thus forming multi-layered photochromic lens. These layers of such photochromic lens are almost integrated in one structure and are not easy to fall off, featuring by reliable and stable. Also, as the photochromic material is placed between the first layer of PC film and the second layer of PC film, it won't be affected even being exposed to the sun and rain, thus the photochromic effect can be maintained for a long time. It can be widely used in helmet lens, ski lens and other lenses requiring photochromic effect.

The present invention will be further explained from the following detailed description taken in conjunction with the accompanying drawings.

### Brief Description of the Drawings

FIG 1 is the overall structural view of embodiment 1;
FIG 2 is the exploded structural view of embodiment 1;
FIG 3 is the overall structural view of embodiment 2;
FIG 4 is the exploded structural view of embodiment 2;
FIG 5 is the overall structural view 1 of embodiment 3;
FIG 6 is the overall structural view 2 of embodiment 3;

In the figure: photochromic material 1; first layer of PC film 2; second layer of PC film 3; helmet lens 4; photochromic sheet 5; mounting hole 6; adjusting structure 7; ski lens 8.

### Detailed Description of the Preferred Embodiments

### Embodiment 1

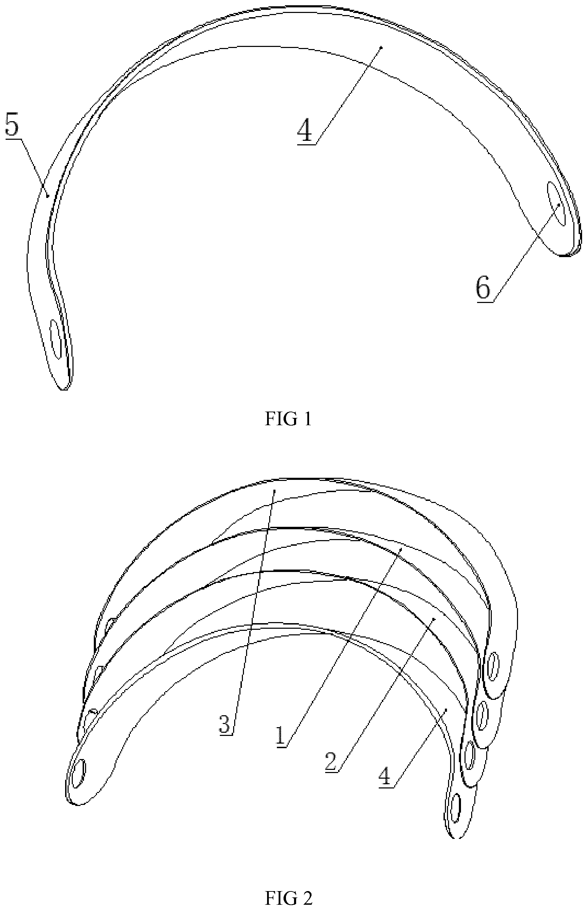

Refer to FIG 1 and FIG 2, the manufacturing method of photochromic helmet lens provided in this embodiment comprises the following steps:
1) Place the photochromic material 1 between the first layer of PC film 2 and the second layer of PC film 3, glue with optical cement and then heat and bend to finalize the design, thus forming photochromic sheet 5 matching with the lateral sides of helmet lens 4; wherein, the said photochromic material 1 is of thin film structure sandwiched between the first layer of PC film 2 and the second layer of PC film 3, or a coating structure sprayed between the first layer of PC film 2 and the second layer of PC film 3; the said photochromic material 1 is a kind of UV photochromic material, and the thickness of the said first layer of PC film 2 and the second layer of PC film 3 is between 0.1mm-0.5mm.
2) Place the photochromic sheet 5 formed in Step 1) in the injection mold cavity of helmet lens in advance, which is placed in the lateral sides of helmet lens;
3) Inject the melted PC material into the cavity. Before curing, the PC material is at a high temperature of 260-280 , under which the PC material and photochromic sheet 5 will fuse into one. After curing, a multi-layered photochromic helmet lens is obtained. Wherein, it is required to note that the above-mentioned high temperature of 260-280 is the temperature of PC material injected in the cavity with pressure after melting in screw melter. It is important to emphasize that the first layer of PC film and the second layer of PC film in Step 1) is necessary, because they are a key link in the whole process. Only after combining the said PC films with the photochromic material to obtain photochromic sheet can adapt to the follow-up intramode injection molding.

A photochromic helmet lens manufactured by the said method, including the helmet lens 4 and the photochromic sheet 5 attached to the lateral sides of helmet lens 4. It is characterized in that the said photochromic sheet 5 is a four-layered structure sheet which is obtained by placing the photochromic material 1 between the first layer of PC film 2 and the second layer of PC film 3 and gluing through optical cement. There are four layers, i.e., first layer of PC film, second layer of PC film, a layer of photochromic material and a layer of optical cement. Wherein, the said photochromic material 1 is a thin film structure sandwiched between the first layer of PC film 2 and the second layer of PC film 3, or, the said photochromic material 1 is a coating structure coated between the first layer of PC film 2 and the second layer of PC film 3. The said photochromic material 1 is a kind of UV photochromic material. The thickness of the first layer of PC film 2 and the second layer of PC film 3 is between 0.1mm-0.5mm.

As the first style, the said helmet lens is provided with a mounting hole 6 on both ends, the inside of the hole 6 is smooth, and the said photochromic sheet 5 fully covers and fuses with the lateral sides of helmet lens 4;

### Embodiment 2

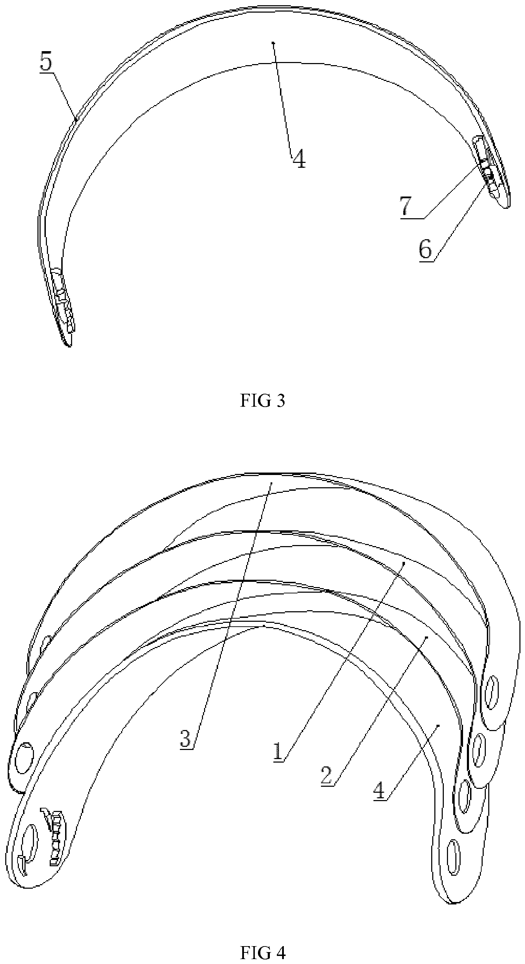

Refer to FIG 3 and FIG 4, this embodiment is basically same with the embodiment 1 and only differs in that:
As the second style, the said helmet lens 4 is provided with a mounting hole 6 on both ends, the inside of the hole 6 is of raised adjusting structure 7, and the said photochromic sheet 5 fully covers and fuses with the lateral sides of helmet lens 4.

### Embodiment 3

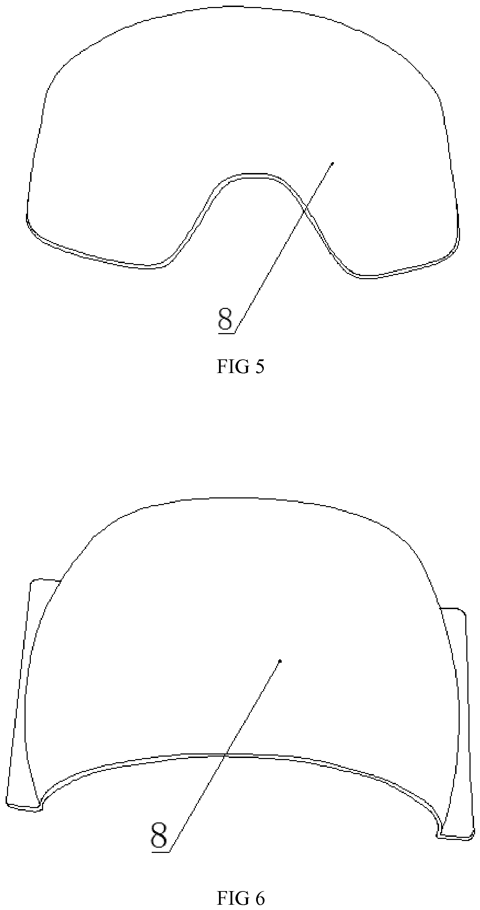

Refer to FIG 5 and FIG 6, this embodiment is basically same with the embodiment 1 and only differs in that: This embodiment provides a method of manufacturing photochromic ski lens and a photochromic ski lens manufacture by the method. That is, replace the helmet lens 4 described in embodiment 1 with the ski lens 8.

The present invention is not limited to above embodiments. Other methods of manufacturing photochromic lenses and their articles of manufacture by adopting same or similar arts with above embodiments of the invention are within the scope of protection of the present invention.

## Claims

1. A method of manufacturing photochromic lens, which is **characterized in that** the method includes the following steps:
1) Put photochromic materials between the first layer of PC film and the second layer of PC film, glue with optical cement, and then finalize the design, thus forming a photochromic sheet matching with the lateral sides of lens;
2) Place the photochromic sheet formed in Step 1) in the injection mold cavity of lens in advance, which is placed in the lateral sides of lens;
3) Inject the melted PC material into the cavity. Before curing, the PC material is at a high temperature of 260-280 , under which the PC material and photochromic sheet will fuse into one. After curing, a multi-layered photochromic lens is obtained.

2. According to the manufacturing method of photochromic lens stated in claim 1, which is **characterized in that**, in step 1), the said photochromic material is a thin film structure sandwiched between the first layer of PC film and the second layer of PC film, or, the said photochromic material is a coating structure coated between the two layers of PC films.

3. According to the manufacturing method of photochromic lens stated in claim 1 or 2, which is **characterized in that**, in step 1), the said photochromic material is a kind of UV photochromic material.

4. According to the manufacturing method of photochromic lens stated in claim 1, which is **characterized in that**, in step 1), the thickness of the said first layer of PC film and second layer of PC film is between 0.1mm-0.5mm.

5. According to the manufacturing method of photochromic lens stated in claim 1, which is **characterized in that**, in step 1), the said photochromic material is placed between the first layer of PC film and the second layer of PC film, glued through optical cement and heated and bended to finalize the design.

6. A photochromic lens manufactured by using any method of claim 1 to 5, including the lens and the photochromic sheet attached to the lateral sides of lens. It is **characterized in that** the said photochromic sheet is a four-layered structure sheet which is obtained by placing the photochromic material between the first layer of PC film and the second layer of PC film and gluing through optical cement.

7. According to the photochromic lens stated in claim 6, which is **characterized in that** the said photochromic material is a thin film structure sandwiched between the first layer of PC film and the second layer of PC film, or, the said photochromic material is a coating structure coated between the two layers of PC films.

8. According to the photochromic lens stated in claim 6 or 7, which is **characterized in that** the said photochromic material is a kind of UV photochromic material.

9. According to the photochromic lens stated in claim 6, which is **characterized in that** the thickness of the said first layer of PC film and second layer of PC film is between 0.1mm-0.5mm.

10. According to the photochromic lens stated in claim 6, which is **characterized in that** the said lens is a kind of helmet lens or ski lens; wherein, the said helmet lens is provided with a mounting hole on both ends, the inside of the hole is smooth, and the said photochromic sheet fully covers and fuses with the lateral sides of helmet lens; or, the said helmet lens is provided with a mounting hole on both ends, the inside of the hole is of raised adjusting structure, and the said photochromic sheet fully covers and fuses with the lateral sides of helmet lens.
